(19) Europäisches Patentamt
European Patent Office
Office européen des brevets

(11) Numéro de publication: **0 001 670**
**B1**

(12) **FASCICULE DE BREVET EUROPEEN**

(45) Date de publication du fascicule du brevet: **13.04.83**

(21) Numéro de dépôt: **78200256.2**

(22) Date de dépôt: **19.10.78**

(51) Int. Cl.³: **C 07 C 17/42,**
C 07 C 19/02, C 09 K 3/30

(54) **Compositions stabilisées comprenant du chlorure de méthyléne.**

(30) Priorité: **24.10.77 FR 7732188**

(43) Date de publication de la demande:
**02.05.79 Bulletin 79/9**

(45) Mention de la délivrance du brevet:
**13.04.83 Bulletin 83/15**

(84) Etats contractants désignés:
**BE CH DE FR GB LU NL SE**

(56) Documents cités:
**FR - A - 2 178 898**
**GB - A - 765 522**

**CHEMICAL ABSTRACTS, vol. 83, 58070c (1975)**
**CHEMICAL ABSTRACTS, vol. 83, 58071d (1975)**

(73) Titulaire: **SOLVAY & Cie (Société Anonyme)**
**Rue du Prince Albert, 33**
**B-1050 Bruxelles (BE)**

(72) Inventeur: **Godfroid, Marcel**
**Rue de Nivelles, 117**
**B - 1300 Wavre (BE)**
Inventeur: **Gerkens, Roger**
**Rue du Cuisinier, 56**
**B - 1420 Braine-L'Alleud (BE)**

(74) Mandataire: **Eischen, Roland**
**Solvay & Cie Département de la Propriété Industrielle Rue de Ransbeek 310**
**B-1120 Bruxelles (BE)**

**EP 0 001 670 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Compositions stabilisées comprenant du chlorure de méthylène

La présente invention concerne des compositions comprenant du chlorure de méthylène stabilisées en vue d'éviter sa décomposition lors de son stockage ou lors de son emploi, et convenant notamment pour des aérosols.

Le chlorure de méthylène est fréquemment utilisé dans les compositions pour aérosols en tant que solvant des matières actives et dépresseur pour le propulseur (dichlorodifluorométhane, butane, propane, dioxyde de carbone, hémioxyde d'azote, etc.). Cependant le chlorure de méthylène a tendance à se décomposer notamment au contact des surfaces métalliques et sous l'influence de la chaleur. La corrosion des surfaces métalliques, qui en résulte, devient particulièrement importante lorsque les compositions pour aérosols contiennent, outre le chlorure de méthylène, de l'eau. Cette agressivité du chlorure de méthylène est notamment très marquée vis-à-vis des métaux fréquemment utilisés dans la fabrication des boîtiers pour aérosols tels que l'aluminium ou l'acier étamé.

On a déjà proposé de pallier la dégradation des surfaces métalliques par les halogénométhanes, en leur ajoutant de petites quantités d'un stabilisant de nature organique. Parmi les stabilisants qui ont été proposés pour stabiliser les méthanes chlorés tels que le chlorure de méthylène, on peut citer diverses substances telles que l'amylène (Br. Etats-Unis 1904450 au nom de DU PONT DE NEMOURS déposé le 3.3.1931), le phénol (Brevet Etats-Unis 2008680 au nom de DU PONT DE NEMOURS déposé le 3.3.1931), les composés nitrés (Brevet britannique 773 187 déposé le 8.12.1955 au nom de FARBWERKE HOECHST AG), le diméthoxyméthane (Brevet belge 741 556 déposé le 12.11.1969 au nom de DOW CHEMICAL Co) et les époxydes (Brevet Etats-Unis 2106158 au nom de I. G. FARBEN déposé le 4.7.1935).

L'addition de ces produits au chlorure de méthylène ne permet cependant pas d'éviter la corrosion des surfaces métalliques lorsque le chlorure de méthylène est utilisé en mélange avec de l'eau.

Pour pallier cet inconvénient on a proposé d'utiliser des mélanges synergiques de stabilisants. Parmi les mélanges de stabilisants qui ont été proposés pour stabiliser le chlorure de méthylène, on peut citer les mélanges de 2-méthylfuranne avec des époxydes (Br. belge 781 796 déposé le 7 avril 1972 au nom de la Demanderesse). Ces mélanges se sont avérés particulièrement efficaces pour stabiliser le chlorure de méthylène en présence de petites quantités d'eau (d'environ quelques pourcents par rapport au chlorure de méthylène). Ils ne permettent cependant pas de conférer au chlorure de méthylène). Ils ne permettent cependant pas de conférer au chlorure de méthylène une stabilité suffisante lorsqu'il est stocké en présence de quantités d'eau plus importantes.

Par ailleurs, selon le brevet britannique GB - A - 765 522 déposé le 7 février 1955 au nom de DIAMOND ALKALI COMPANY, il est connu de stabiliser les hydrocarbures chlorés à faible poids moléculaire tels que le trichloroéthylène et tétrachloroéthylène, en même temps que les sous-produits qu'ils contiennent, habituellement (dichloroéthylène, trichloroéthylène, trichloroéthane, tétrachloroéthane, pentachloro-éthane et les analogues), au moyen de composés organiques d'espèces trés nombreuses et très diverses. Les amines figurent parmi ces espèces et l'hexaméthylènetétramine est mentionnée comme exemple d'amine.

La présente invention vise à résoudre le problème de la stabilisation du chlorure de méthylène en présence de quantités d'eau importantes.

La présente invention concerne à cet effet des compositions comprenant comme composant de base du chlorure de méthylène stabilisées avec un mélange de stabilisants comprenant au moins un époxyde et un autre stabilisant dans lesquelles cet autre stabilisant est l'hexaméthylènetétramine.

L'hexaméthylènetétramine (urotropine) répond à la formule

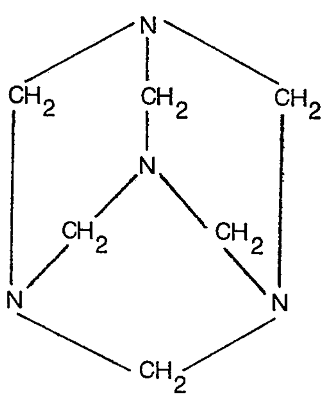

Elle est en général mise en oeuvre selon l'invention à raison de 0,0001 à 2% et le plus souvent de 0,001 à 1% en poids du chlorure de méthylène présent dans la composition.

Comme époxydes on choisit de préférence les époxydes contenant de 2 à 8 atomes de carbone.

Ces époxydes peuvent être substitués, par exemple par des halogènes, des fonctions éthers-oxydes ou des groupes hydroxyles. On utilise le plus souvent des époxydes choisis parmi l'oxyde d'éthylène, l'oxyde de propylène, l'épichlorhydrine, le glycidol, les oxydes de butènes, les oxydes de pentènes, l'oxyde de cyclohexène, l'oxyde de styrène et les méthyl-, éthyl-, propyl-, isopropyl- et tert-butyl-glycidyléthers. Les époxydes vicinaux contenant de 2 à 5 atomes de carbone conviennent particulièrement bien. Parmi ceux-ci on préfère utiliser l'oxyde de propylène, les oxydes de butène, le glycidol, le méthylglycidyléther, l'éthylglycidyléther et l'épichlorhydrine. L'oxyde de propylène, les oxydes de butène, le glycidol et le méthylglycidyléther se sont révélés particulièrement adéquats.

Les époxydes sont en général mis en oeuvre selon l'invention, à raison de 0,001 à 10% et le plus souvent de 0,01 à 5% en poids du chlorure de méthylène à traiter.

L'hexaméthylènetétramine et l'époxyde, sont mis en oeuvre en général dans un rapport pondéral compris entre 0,0001 et 1 et le plus souvent entre 0,001 et 0,2. Des rapports différents peuvent bien entendu être également utilisés.

Il va de soi que l'emploi d'hexaméthylènetétramine, en mélange avec un ou plusieurs époxydes, faisant l'objet de la présente invention peut être combiné avec l'emploi d'un ou plusieurs autres stabilisants connus par ailleurs.

On peut ainsi avantageusement ajouter au chlorure de méthylène stabilisé selon l'invention un ou plusieurs agents antioxydants tels que les alcools et les alkanes, cycloalkanes, alkènes et cycloalkènes non substitués. De façon avantageuse ces agents antioxydants sont ajoutés au chlorure de méthylène stabilisé par l'hexaméthylènetétramine en mélange avec au moins un époxyde.

Comme alcools on choisit de préférence les alcools contenant de 1 à 3 atomes de carbone tels que le méthanol, l'éthanol, le propanol et l'isopropanol. L'éthanol s'est révélé particulièrement adéquat.

Les alcools sont en général mis en oeuvre à raison de 0,01 à 5% et le plus souvent de 0,05 à 3% en poids du chlorure de méthylène à traiter.

Comme alkanes, cycloalkanes, alkènes et cycloalkènes non substitués on choisit de préférence ceux qui contiennent de 5 à 9 atomes de carbone tels que le n-pentane, l'isopentane, le triméthylpropane, les diméthylbutanes, les diméthylpentanes, le triméthylbutane, le cyclohexane, le cyclopentane, le méthylcyclopentane, l'éthylcyclopentane, le méthylcyclohexane, les pentènes, les diméthylbutènes, les méthylbutènes, le diisobutylène, les hexènes, les méthylpentènes, le tripropylène, le cyclopentène, le cyclohexène et le méthylcyclopentène. Les alkanes, cycloalkanes, alkènes et cycloalkènes ayant un point d'ébullition compris entre 0 et 120°C conviennent bien. Parmi ceux-ci on préfère utiliser les pentènes, le cyclohexane et le n-pentane.

Les alkanes, cycloalkanes, alkènes et cycloalkènes sont en général mis en oeuvre à raison de 0,0001 à 2% et le plus souvent de 0,001 à 1% en poids du chlorure de méthylène à traiter.

La quantité totale de stabilisants et agents anti-oxydants mise en oeuvre est en général comprise entre 0,01 et 15% et le plus souvent entre 0,05 et 10% en poids du chlorure de méthylène présent dans la composition.

Les doses des différents stabilisants et agents antioxydants données ci-dessus sont les doses les plus généralement utilisées. Des doses inférieures peuvent être utilisées mais elles sont souvent moins efficaces. Des doses supérieures peuvent également être utilisées, mais elles ne se justifient en général pas et elles présentent peu d'intérêt d'un point de vue économique.

L'invention peut également s'appliquer à la stabilisation de mélanges contenant du chlorure de méthylène.

Le chlorure de méthylène stabilisé suivant l'invention s'est révélé particulièrement stable et peut avantageusement être utilisé dans les compositions pour aérosols à teneur en eau importante pouvant aller sans inconvénient jusqu'à cinq fois le poids de chlorure de méthylène. Le plus souvent, ces compositions contiennent des quantités d'eau comprise entre 5 et 200% en poids de la quantité de chlorure de méthylène mise en oeuvre.

L'exemple suivant, qui n'a aucun caractère limitatif, montre les résultats remarquables obtenus selon un mode de réalisation de l'invention.

Exemple

Afin d'évaluer l'influence du stabilisant faisant l'objet de la présente invention sur du chlorure de méthylène utilisé comme dépresseur dans les boîtiers pour aérosols contenant des quantités importantes d'eau on a comparé l'agressivité d'un mélange homogène contenant un propulseur (propane), un tiers solvant (éthanol), de l'eau et du chlorure de méthylène non stabilisé (essai 1R) ou stabilisé au moyen d'un époxyde (essais 3R et 5R) uniquement à celle du même mélange contenant du chlorure de méthylène stabilisé au moyen d'hexaméthylènetétramine seule ou en mélange avec un époxyde (essais 2, 4 et 6).

L'essai consiste à introduire dans des boîtiers pour aérosols en fer étamé à couture électrique fabriqués par SOBEMI de 280 $cm^3$ de capacité une composition contenant 5% en poids de propane, 42% en poids d'éthanol, 16% en poids d'eau et 37% en poids de chlorure de méthylène stabilisé ou non. Les boîtiers sont remplis à 75% de leur capacité; ils sont disposés verticalement et conservés à 37°C. On observe la corrosion générale de l'intérieur du boîtier après deux mois.

Le tableau I ci-après donne les corrosions observées après deux mois, lors de l'emploi des différentes formules de stabilisation. Le chiffre 1 indique que le boîtier a un aspect identique à celui du boîtier neuf (aucune attaque); 2 indique qu'il y a une faible attaque généralisée; 3 qu'il y a une forte attaque généralisée; 4 qu'il y a quelques piqûres et 5 qu'il y a de nombreuses piqûres (la lettre r indique la présence de taches de rouille). Les formules portant la lettre R sont données à titre de comparaison.

Les quantités de stabilisants mises en oeuvre sont exprimées par rapport au volume de chlorure de méthylène.

TABLEAU I

| Essai | Stabilisants | Importance de la corrosion |
|---|---|---|
| 1R | néant | 2 + 5r |
| 2 | hexaméthylènetetramine 0,1 g/l | 4 à 5r |
| 3R | 1,2-époxypropane 2 g/l | 2 + 5r |
| 4 | hexaméthylènetétramine 0,1 g/l + 1,2-époxypropane 2 g/l | 1 |
| 5R | 1,2-époxybutane 2 g/l | 2 + 5r |
| 6 | hexaméthylènetétramine 0,1 g/l + 1,2-époxybutane 2 g/l | 1 |

Dans tous les cas les corrosions observées se présentent sur le métal en contact avec la phase liquide. Dans aucun cas on n'a observé de corrosion en phase vapeur.

L'examen du Tableau I montre que en présence de grandes quantités d'eau le chlorure de méthylène non stabilisé (essai 1R) ou stabilisé par de l'époxypropane (essai 3R) ou de l'époxybutane (essai 5R) provoque une corrosion importante de boîtiers. Par contre lorsqu'on ajoute de très petites quantités d'hexaméthylènetétramine on observe déjà une réduction sensible de la corrosion (essai 2). L'addition simultanée d'époxyde et de petites quantités d'hexaméthylènetétramine permet de supprimer complètement la corrosion.

**Revendications**

1. Compositions comprenant comme composant de base du chlorure de méthylène, stabilisées avec un mélange de stabilisants comprenant au moins un époxyde et un autre stabilisant, caractérisées en ce que cet autre stabilisant est l'hexaméthylènetétramine.

2. Compositions suivant la revendication 1, caractérisées en ce que les compositions contiennent une quantité d'hexaméthylènetétramine comprise entre 0,0001 et 2% en poids du chlorure de méthylène.

3. Compositions suivant les revendications 1 ou 2, caractérisées en ce que l'époxyde est choisi parmi les époxydes contenant de 2 à 8 atomes de carbone.

4. Compositions suivant la revendication 3, caractérisées en ce que l'époxyde est choisi parmi l'oxyde de propylène, les oxydes de butène, le glycidol et le méthylglycidyléther.

5. Compositions suivant l'une quelconque des revendications 1 à 4, caractérisées en ce qu'elles contiennent en outre un agent antioxydant choisi parmi les alcools et les alkanes, cycloalkanes, alkènes et cycloalkènes non substitués.

6. Compositions suivant la revendication 5, caractérisées en ce que l'alcool est l'éthanol.

7. Compositions suivant la revendication 5, caractérisées en ce que les alkanes, cycloalkanes, alkènes et cycloalkènes non substitués sont choisis parmi le n-pentane, le cyclohexane et les pentènes.

8. Compositions suivant l'une quelconque des revendications 1 à 7, contenant en outre de l'eau.

9. Compositions suivant la revendication 8, contenant de l'eau en quantité pouvant aller jusqu'à 5 fois le poids de chlorure de méthylène.

**Patentansprüche**

1. Als Grundbestandteil Methylenchlorid enthaltende Zusammensetzungen stabilisiert mit einer Mischung von Stabilisatoren enthaltend wenigstens ein Epoxyd und einen anderen Stabilisator, dadurch gekennzeichnet, daß der andere Stabilisator Hexamethylentetramin ist.

2. Zusammensetzungen gemäß Anspruch 1, dadurch gekennzeichnet, daß sie eine Menge an Hexamethylentetramin von zwischen 0,0001 und 2 Gew.-% bezogen auf Methylenchlorid enthalten.

3. Zusammensetzungen gemäß Ansprüchen 1 oder 2, dadurch gekennzeichnet, daß das Epoxyd ausgewählt ist unter den Epoxyden mit 2 bis 8 Kohlenstoffatomen.

4. Zusammensetzungen gemäß Anspruch 3, dadurch gekennzeichnet, daß das Epoxyd ausgewählt ist unter dem Oxyd von Propylen, den Oxyden von Buten, dem Glycidol und dem Methylglycidyläther.

5. Zusammensetzungen gemäß einem der Ansprüche 1 bis 4, dadurch gekennzeichnet, daß sie außerdem ein Antioxydationsmittel enthalten, ausgewählt unter den Alkoholen und den nicht-substituierten Alkanen, Cycloalkanen, Alkenen und Cycloalkenen.

6. Zusammensetzungen gemäß Anspruch 5, dadurch gekennzeichnet, daß der Alkohol Äthanol ist.

7. Zusammensetzungen gemäß Anspruch 5, dadurch gekennzeichnet, daß die nicht-substituierten Alkane, Cycloalkane, Alkene und Cycloalkene ausgewählt sind unter n-Pentan, Cyclohexan und den Pentenen.

8. Zusammensetzungen gemäß einem der Ansprüche 1 bis 7, enthaltend außerdem Wasser.

9. Zusammensetzungen gemäß Anspruch 8, enthaltend Wasser in einer Menge bis zur 5-fachen Gewichtsmenge des Methylenchlorids.

**Claims**

1. Compositions comprising methylene chloride as the base component and stabilised with a mixture of stabilisers comprising at least one epoxide and one other stabiliser, characterised in that this other stabiliser is hexamethylenetetramine.

2. Compositions according to Claim 1, characterised in that they contain an amount of hexamethylenetetramine of between 0.0001 and 2% by weight of the methylene chloride.

3. Compositions according to Claims 1 or 2, characterised in that the epoxide is chosen from amongst epoxides containing from 2 to 8 carbon atoms.

4. Compositions according to Claim 3, characterised in that the epoxide is chosen from amongst propylene oxide, butene oxides, glycidol and methyl glycidyl ether.

5. Compositions according to any one of Claims 1 to 4, characterised in that they also contain an antioxidant chosen from amongst alcohols and unsubstituted alkanes, cycloalkanes, alkenes and cycloalkenes.

6. Compositions according to Claim 5, characterised in that the alcohol is ethanol.

7. Compositions according to Claim 5, characterised in that the unsubstituted alkanes, cycloalkanes, alkenes and cycloalkenes are chosen from amongst n-pentane, cyclohexane and pentenes.

8. Compositions according to any one of Claims 1 to 7, which also contain water.

9. Compositions according to Claim 8, which contain water in an amount which can range up to 5 times the weight of methylene chloride.